(19) **Europäisches Patentamt / European Patent Office / Office européen des brevets**

(11) **EP 2 070 468 A1**

(12) **EUROPEAN PATENT APPLICATION**

(43) Date of publication:
**17.06.2009 Bulletin 2009/25**

(51) Int Cl.:
**A61B 5/00** *(2006.01)* **G02B 3/12** *(2006.01)*

(21) Application number: **07123234.2**

(22) Date of filing: **14.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Koninklijke Philips Electronics N.V.**
**5621 BA Eindhoven (NL)**

(72) Inventor: **The designation of the inventor has not yet been filed**

(74) Representative: **Gravendeel, Cornelis**
**Philips**
**Intellectual Property & Standards**
**P.O. Box 220**
**5600 AE Eindhoven (NL)**

(54) **An optical image probe**

(57) The present invention relates to an optical image probe (20) particular suited for optical coherence tomography (OCT). The probe comprises an optical light guide (1) arranged for a transmitting single-mode light and a fluid lens (5) positioned at an end portion of the optical light guide, the fluid lens having a changeable numerical aperture (1NA). Additionally, a lens (10) is positioned in front of the fluid lens, the lens having a fixed numerical aperture (2NA). The invention is advantageous for obtaining an optical image probe that facilitates a simultaneously change in the focal position so that the sample point may coincide with a position within the focal depth of the lens system of the optical image probe. This will in turn allows high-resolution depth scan in combination with a high lateral resolution. Previously, a balance between focal depth and lateral resolution had to be made with OCT. This is avoided by the present invention whereby a satisfactory focal depth may be achieved simultaneously with a lateral or transverse resolution hitherto not possible.

20:

FIG. 1

## Description

FIELD OF THE INVENTION

The present invention relates to an optical image probe, the probe is particular suited for miniature application e.g. in-vivo. The invention also relates to an optical coherence tomography (OCT) with the said optical image probe and a corresponding method for imaging.

BACKGROUND OF THE INVENTION

Optical Coherence Tomography (OCT) is an emerging imaging technology, especially in medical imaging. It achieves up to a few millimeters' penetration depth (1.5-2mm typically) at ultrahigh resolution (several microns) generating 3D tissue images in real time. OCT provides 3D structural image (tissue layers, density changes), showing currently great potential to provide spectroscopic information and to achieve functional and molecular imaging as well. Optical Coherence Tomography (OCT) is typically based on a low time-coherence Michelson interferometer i.e. time-domain OCT, but frequency-domain OCT is also possible. OCT synthesizes cross-sectional images from a series of laterally adjacent depth-scans.

In time-domain OCT, one arm serves as a reference arm of the interferometer, while the other one delivers light to the sample (sample arm). The scanning optics provides lateral scanning capabilities, so that the OCT setup obtains one axial-scan for each lateral position. All axial-scans combined form a 3D structural image. When obtaining each axial-scan the reference mirror's displacement provides depth information. The lateral resolution is determined by the numerical aperture (NA) of the lens in the sample arm. The numerical aperture can not be too large because the depth scan can only give high resolution within the focus depth of the above mention lens. A high NA would mean small focal depth. Accordingly, most OCT imaging systems have a relatively low numerical aperture (NA) in order to have sufficiently large depth of focus, but this will be at the expense of the lateral or transverse resolution of the OCT imaging system. For further reference on the resolution limits of OCT, the reader is referred to Handbook of Optical Coherence Tomography, edited by Bouma and Tearney, Introduction by Fujimoti, p. 6-8, 2002.

One way of solving this problem is by adjusting the position of the focal point of the lens in the sample arm for instance by mechanical displacement. When using OCT inside the human body during minimal invasive procedures, for instance by bringing the sample arm inside the body through the working channel of an endoscope, the mechanical way of focusing is not suitable because of the small dimensions required.

Hence, an improved optical image probe would be advantageous, and in particular a more efficient and/or reliable optical image probe would be advantageous.

SUMMARY OF THE INVENTION

It may be seen as an object of the present invention to provide an optical probe that seeks to mitigate, alleviate or eliminate one or more of the above mentioned disadvantages, singly or in any combination, the above mentioned problems of the prior art with the inherent compromise between focus depth and transverse/lateral resolution for optical coherence tomography (OCT).

Thus, the above described object and several other objects are intended to be obtained in a first aspect of the invention by providing an optical image probe, the probe comprising:

- an optical light guide arranged for a transmitting single-mode light,
- a fluid lens positioned at an end portion of the optical light guide, the fluid lens having a changeable numerical aperture (1NA), and
- a lens positioned in front of the fluid lens, the lens having a fixed numerical aperture (2NA).

The invention is particularly, but not exclusively, advantageous for obtaining an optical image probe facilitating a simultaneously change in the focal position so that the sample point may coincide with a position within the focal depth of the lens system of the optical image probe. This will in turn allows high-resolution depth scan in combination with a high lateral resolution.

The previous compromise or balance between focal depth and lateral resolution can accordingly be lifted or avoided by the present invention whereby a satisfactory focal depth may be achieved simultaneously with a lateral or transverse resolution hitherto not possible. Thus, the present invention may enable OCT imaging with a focal depth of several millimeters and, at the same time, a lateral resolution below 10 micrometer, possible below 5 micrometer and even further down. This opens up new possible applications for OCT.

Preferably, the fixed numerical aperture (2NA) may be larger than a maximum value of the changeable numerical aperture (1NA) so to provide a sufficient numerical aperture or optical power to have both high focal depth and high lateral resolution. More particularly, the numerical aperture (2NA) of the lens and the numerical aperture (1NA) of the fluid lens may in combination facilitate a lateral resolution of the optical probe below 10 micrometer, preferably below 8 micrometer, or more preferably below 6 micrometer. This may, in turn, define a wave length interval as it will be appreciated. Specifically, the lens in front of the fluid lens may have a numerical aperture (NA) of at least 0.3, at least 0.5, or at least 0.7. Other limits will be discussed at more length in the detailed description below.

In one embodiment, the fluid lens may be an electrowetting lens comprising two immiscible fluids, e.g. two liquids such as oil and water. More particularly, the electrowetting lens may have an asymmetric electrode configuration so as to enable tilting of the meniscus formed between the two immiscible fluids. Thus, with this embodiment one may "bend" the optical axis. This may be quite useful for in-vivo applications.

Preferably, the probe may be arranged for optical coherence tomography (OCT), confocal imaging, spectroscopy imaging, or fluorescent imaging. Other kind of spectroscopy and/or imaging is also contemplated within the teaching of the present invention.

In another embodiment, the optical image probe may be arranged to form part of an endoscope, a catheter or a needle. Thus, in-vivo application requires miniaturization in general and therefore the present invention may have a particular advantage in such fields.

In yet another embodiment, the probe may be arranged for optical coherence tomography (OCT), and the probe may have a corresponding reference arm. Typically, the probe may itself be embedded in a so-called sample arm. The reference arm may then have substantially the same optical properties as the optical image probe at least with respect to optical dispersion and intensity loss. Thus by "matching" the probe with the reference arm, which may be manufactured and/or sold together with the optical probe, it is possible to ensure a sufficient OCT image quality. Typically, the reference arm will also not be integrated into a permanent position of the imaging system, because it must substantially match with respect to at least dispersion and intensity losses as compared to that of the optical image probe i.e. the sample arm.

In a second aspect, the present invention relates to an imaging system for optical coherence tomography (OCT), the system comprising:

- a light source,
- a sample arm optically coupled to the light source, the sample arm having at its distal end an optical image probe according to the first aspect, and
- an imaging detector optically coupled to the light source and the sample arm.

This aspect of the invention is particularly, but not exclusively, advantageous for obtaining an imaging system that facilitates a parameter related to axial scan i.e. reference mirror or tunable wavelength may be changed while the numerical aperture (NA) of the optical image probe in the sample arm is being changed accordingly so to keep the both focal depth and the transverse resolution at a satisfactory level.

In one embodiment, the system may be arranged for time-domain optical coherence tomography (TD-OCT), the imaging detector may further be optically coupled to a reference arm with a displaceable reference mirror. The numerical aperture (1NA) of the fluid lens may then be dependent on the position of the reference mirror during axial scans of the imaging system. Thus, the numerical aperture (1NA) may follows or change accordingly with respect to the displacement of reference mirror.

In another embodiment, the system may be arranged for frequency-domain optical coherence tomography (FD-OCT), the light source may have a tunable wavelength. Then the numerical aperture (1NA) of the fluid lens may be dependent on the wavelength of the light source.

For both kind of OCT (i.e. FD-OCT and TD-OCT), the system may be adapted to adjust the numerical aperture (1NA) of the fluid lens so as to keep the sampling point (SP) within the focal dept (F) of the imaging system. Thus, the numerical aperture (1NA) may change so as to sample within the focal depth. This may result in a significant improvement in possible lateral resolution.

In a third aspect, the present invention relates to a method for imaging with an imaging system for optical coherence tomography (OCT), the method comprising:

- providing a light source (LS),
- providing a sample arm optically coupled to the light source, the sample arm having at its distal end an optical image probe according to the first aspect, and
- performing imaging with an imaging detector (ID) optically coupled (C) to the light source (LS) and the sample arm.

The individual aspects of the present invention may each be combined with any of the other aspects. These and other aspects of the invention will be apparent from the following description with reference to the described embodiments.

BRIEF DESCRIPTION OF THE FIGURES

The invention will now be described in more detail with regard to the accompanying figures. The figures show one way of implementing the present invention and is not to be construed as being limiting to other possible embodiments falling within the scope of the attached claim set.

Figure 1 is a schematic cross-sectional view of an optical image probe according to the present invention,
Figure 2 is a schematic diagram of an imaging system for optical coherence tomography (OCT) according to the present invention,
Figure 3 is a schematic diagram of an imaging system for time-domain optical coherence tomography (TD-OCT) according to the present invention,
Figure 4 is a schematic cross-sectional view of an optical image probe with asymmetric electrode configuration according to the present invention,
Figure 5 shows two schematic cross-sectional views of an optical image probe according to the present invention, and

Figure 6 is a schematic flow chart of a method for imaging according to the present invention.

## DETAILED DESCRIPTION OF AN EMBODIMENT

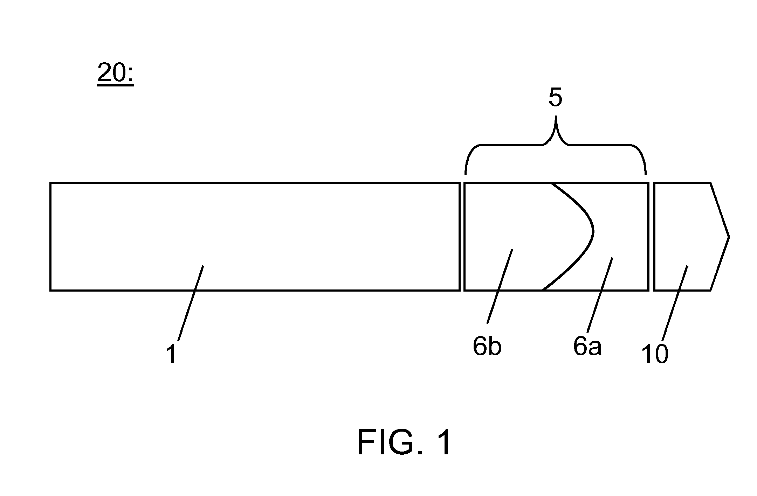

Figure 1 is a schematic cross-sectional view of an optical image probe 20 according to the present invention. The probe comprises an optical light guide 1 arranged for a transmitting single-mode light i.e. a light guide designed to carry one ray of light but typically with a variety of wavelengths. The fiber is thus designed for enabling coherence detection. Such single-mode fibers are quite suitable for OCT because of the low level of dispersion, strictly speaking distortion. Accordingly, the single-mode fiber may also have a broad bandwidth as compared to multi-mode fibers. In particular, the single-mode fiber may be a photonic crystal fiber with a hollow core. Polarization maintaining fibers are also a possibility for the single-mode fiber.

The probe 20 also comprises a fluid lens 5 positioned at an end portion of the optical light guide 1. The fluid lens 5 has a changeable numerical aperture (1NA). Preferably, the fluid lens 5 is an electrowetting lens comprising two immiscible fluids 6a and 6b, e.g. two liquids such as an oil and water. Further details of the electrowetting lens may be found in US patent application (to the same applicant) US 20050113912, which is hereby incorporated by reference in its entirety. The fluid lens may have upper and a lower numerical aperture (1NA) depending on the working operations of the lens 5.

The probe 20 further comprises a lens 10 positioned in front of the fluid lens 5, the lens 10 having a second, fixed numerical aperture (2NA), e.g. the lens 10 may a manufactured in a suitable glass or polymer. The lens 10 is preferably a high NA lens. For example, the lens 10 in front of the fluid lens may have a minimum numerical aperture (2NA) of approximately 0.3, 0.4, 0.5, 0.6, 0.7, 0.8, 0.9, 1.0 or >1.0. When the object under study is not in air but in a fluid like water (as may be the case for instance inside the body; in-vivo) the numerical aperture 2NA can be larger than 1.0 because of the equation; 2NA= n sin(theta), and hence when the refractive index, n, of the medium is >1.0 the numerical aperture (2NA) can also be larger than 1.0. Thus, the lens 10 can alternatively have a minimum numerical aperture (2NA) of approximately 1.1, 1.2, 1.3, 1.4, 1.5 or even higher.

The numerical aperture (2NA) of the lens 10 should be designed so as to match the working range of the OCT imaging system, in particular the scanning parameters relating to the resolution, specifically the lateral or transverse resolution and the focal depth.

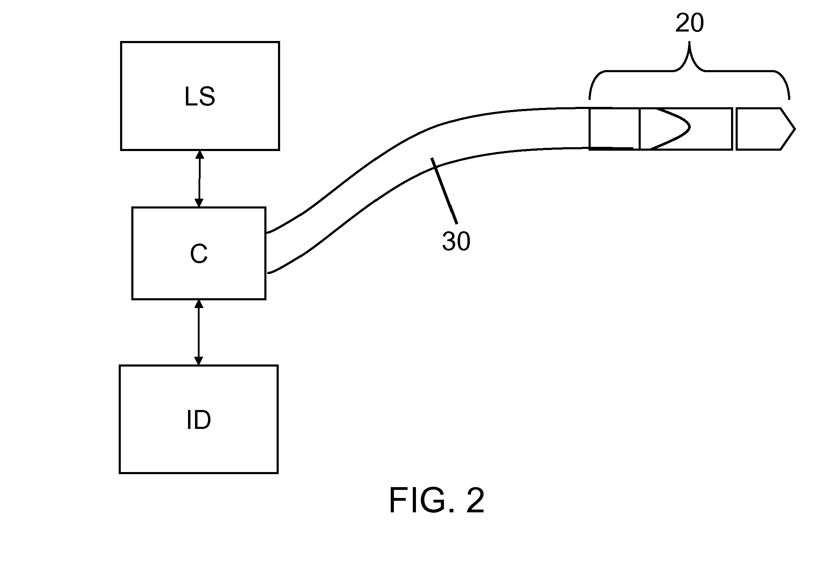

Figure 2 is a schematic diagram of an imaging system for optical coherence tomography (OCT) according to the present invention. The imaging system for optical coherence tomography (OCT) comprises a light source LS, e.g. a laser or an LED suitable for OCT, and a sample arm optically coupled to the light source LS via a coupler C. The sample arm has at its distal end an optical image probe 20. Additionally, the system has an imaging detector ID optically coupled to the light source and the sample arm.

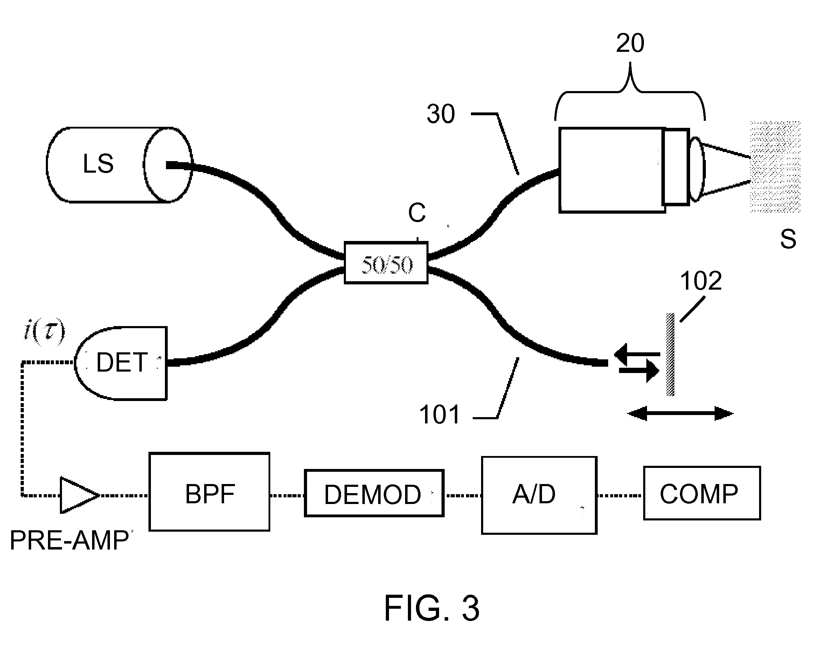

Firstly, the imaging system may be arranged for time-domain optical coherence tomography (TD-OCT). Thus, the imaging detector ID is optically coupled to a reference arm with a displaceable reference mirror, cf. Figure 3. The numerical aperture (1NA) of the fluid lens 5 is then dependent on the position of the reference mirror during axial scans of the imaging system. Thus, the numerical aperture (1NA) effectively follows the displacement of a reference mirror 102, cf. Figure 3.

Secondly, the system may be arranged for frequency-domain optical coherence tomography (FD-OCT), where the light source LS has a tunable wavelength (i.e. swept source of time encoded; FD-OCT), the numerical aperture (1NA) of the fluid lens 5 then being dependent on the wavelength of the light source LS.

In both of these variants of the imaging system according to the present invention, the system is adapted to adjust the numerical aperture (1NA) of the fluid lens 5 so as to keep the sampling point (SP) within the focal dept (F) of the imaging system. Thus, the numerical aperture (1NA) is changed so as to sample within the focal depth or distance (F). This results in a significant improvement in the lateral resolution concurrently with achieving an acceptable focal depth.

Other kind of optical imaging techniques, apart from OCT, may also benefit from an optical image probe according to the present invention. Thus, it is contemplated that confocal imaging, spectroscopy imaging, and possible fluorescent imaging may apply an optical image probe according to the present invention.

Figure 3 is a schematic diagram of an imaging system for time-domain optical coherence tomography (TD-OCT) according to the present invention with similar annotation as Figure 2. The imaging system performs an imaging of the sample S. The so-called sample arm 30 has, at an end portion, an optical image probe 20 according to the present invention, the probe 20 comprising a fluid lens 5 with changeable numerical aperture (1NA) and another lens (with fixed numerical aperture, 2NA) in front thereof.

For TD-OCT, the imaging system requires a so-called reference arm 101, the reference arm comprising a displaceable reference mirror 102 so as to perform axial scannings as indicated by the double-headed arrow.

The image detector ID shown in Figure 2 may accordingly comprise a photosensitive detector DET with a fast response time, where the signal can be pre-amplified in the amplifier PRE-AMP and bandpass-filtered in the filter BPF. After appropriate demodulation in the unit DEMOD and A/D conversion in the converter A/D (both may be part of the image detector ID), the result may be accessed/analyzed on a computer COMP with suitable computer program (software) therefore.

The light source LS, the sample arm 30, the reference arm 101, and the image detector ID are optically coupled together via the fiber coupler C. The coupler may be a 50/50 coupler, where 50% of the light from the light source LS will enter sample arm 30 and the 50% will go to the reference arm 101

The fluid lens 5 is on the side facing the sample S (body tissue) and is covered by a high-NA lens 10 as indicated in Figure 3. Additional driving means (not shown) are present such that displacement of the reference mirror 102 generates a driving signal that changes the numerical aperture (1NA) of the fluid lens correspondingly. To select the right setting, a look-up table can for example be used.

To show that it is possible to change the focal length over a sufficient distance to keep the sample point within the focal depth (F_D) during an axial scan over 1mm, an example will be given below. Typical parameters may be:

**Table 1**

| Entrance pupil diameter | 5mm |
|---|---|
| Numerical aperture (NA) | 0.85 |
| Focal length F | 2.94 mm |
| Wavelength ($\lambda$) | 650 nm |
| Focal depth D (1.22 $\lambda$ /NA$^2$) | 1.10 microm |
| Spot size S (1.22 $\lambda$ /NA) | 0.93 microm |
| Scan range L | +/- 0.5 mm |

The combined focal length F of the fluid lens 5 and fixed lens 10 is given by equation (1);

$$\frac{1}{F} = \frac{1}{f_{liquid}} + \frac{1}{f_{fixed}} \qquad (1)$$

with $f_{fixed}$=2.94mm and $f_{fluid}$= $\infty$ (infinity). In order to change the focal length F = 2.94 mm of the fixed lens 10 in combination with the electowetting lens 5 by 0.5mm, the focal length f of the fluid lens 5 has to change from $\infty$ (infinity) to $f_{fluid}$=-20.23 mm (hence then F=3.44mm). With $\Delta$n=0.2 for the fluids one finds that the radius of the meniscus in the fluid lens 5 must be -4.0mm. This is possible with for instance a fluid lens 5 having an inner diameter cylinder radius of 2.5 mm.

From Handbook of Optical Coherence Tomography, edited by Bouma and Tearney, Introduction by Fujimoti, p. 6-8, 2002, the axial resolution can be found as

$$\Delta z = \frac{2\ln 2}{\pi}\left(\frac{\lambda^2}{\Delta\lambda}\right) \qquad (2)$$

and using the relation d=2f NA, the focal depth can found as

$$b = \frac{2\lambda}{\pi NA^2} \qquad (3)$$

For conventional OCT it is typically the case that the width that can axially be resolved, $\Delta z$, is smaller than the focal depth b; i.e. $\Delta z$ < b.

Taking again an illustrating example with typical OCT parameters; wavelength ($\lambda$) 1300nm and a wavelength change (FWHM of the power width for a Gaussian beam, $\Delta\lambda$) of 70 nm, the axial resolution can be found to be $\Delta z$= 10 micrometers. Thus, the inequality b> $\Delta z$ yields a limit on the numerical aperture of NA<0.28, which in turn limits the transverse resolution, cf. **Table 1.**

However, with the present invention this limitation is lifted, and the axial resolution may be comparable or larger than the focal depth. Thus, in order to have a significant depth scan refocusing is required by changing the numerical aperture (1NA) of the fluid lens 5. Hence for this case, the inequality may be b<= $\Delta z$.

In general, without the present invention the scan depth, often defined as Q, will be chosen to be of the order of the focal depth b (Q<b). However, with the present invention one may choose that the scan depth is larger than or equal to the focal depth; Q>=b.

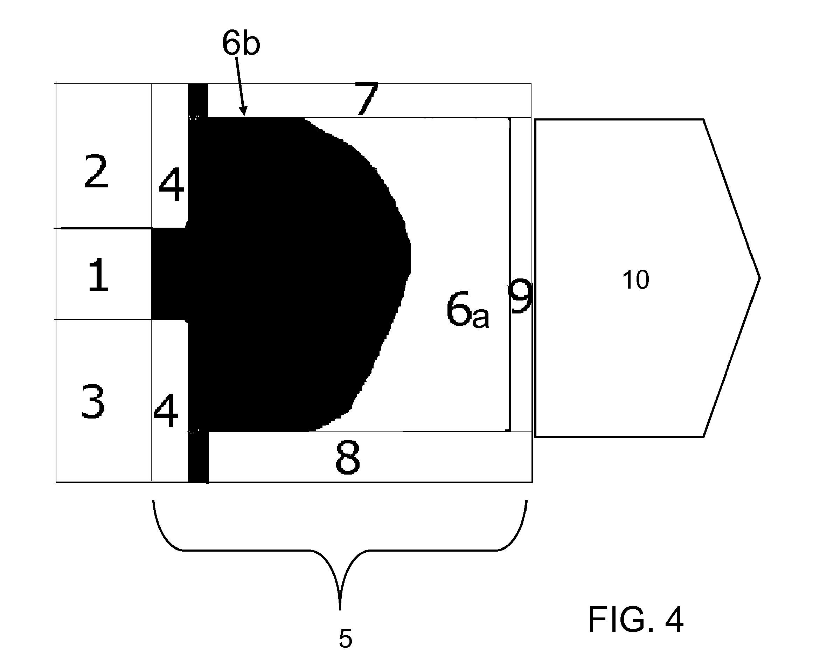

Figure 4 is a schematic cross-sectional view of an optical image probe with asymmetric electrode configuration according to the present invention. In order to minimize the fiber-outcoupling losses, one has to make sure that the refractive index of the first fluid 6b and that of the core of the fiber 1 are as close as possible. When the end part of the fiber (specifically the cladding) is *n*-type doped such that is becomes electrically active (e.g. by incorporation sufficient quantities of arsenic, phosphorous or another group V element), the electrode 4 may be removed. In this case, contacting of the bottom electrode 4 can occur outside of the fiber.

Another way to contact liquid 6b from outside is to evaporate a conducting layer on the outside of the cladding. Due to the index-matching between the core 1 and the liquid 6b, nearly 100% of all light coupled into the fiber will end up in the first liquid 6b. The cladding of the core 1 is rotationally symmetric i.e. 2 and 3 describe the same material. The material should have a lower reflective index than the core 1 to ensure light-guiding. In order to maintain a good mode profile immediately after outcoupling from the core into the first liquid 6b, the refractive index of the cladding 2 and 3 are chosen to be the same as that of the electrode 4.

The second liquid 6a should have a different refractive index as compared to the first liquid 6b in order to allow for lens-action when the meniscus between the two liquids is curved. The two liquids are required to be immiscible to obtain a stable meniscus. By suitable choice of the surface tension between the two liquids and the geometry of the cavity (cylindrical or conical), both concave and convex lens action can be obtained.

The electrodes on the sides as indicated by 7 and 8 could be a series of electrodes i.e. more than the two electrodes drawn for simplicity here, that are equidistantly spaced on the circumference of the lens. In order to prevent short-circuits, the side electrodes do not run all the way to the bottom electrode 4 as indicated by the black liquid between electrode 4 and electrode 7 and 8, respectively. By applying the same voltage on all electrodes, a spherical meniscus can be obtained as a special case. However, by suitable voltage differences between these electrodes, the plane of the meniscus can be tilted as desired. This will result in a directionality of the focal point of the lens, allowing e.g. a surgeon to focus on objects that are not directly in front of the lens system. Such a tilted meniscus will also enable a surgeon to illuminate around a corner during an in vivo inspection.

Due to the finite number of electrodes placed around the two fluids 6a and 6b, no perfectly tilted meniscus will be possible. Therefore, aberrations and wave front errors will be introduced to some extent.

Finally, an optically transparent cover layer 9 is placed on top of the lens in order to prevent liquid leaking out of the liquid lens 5. Adjacent to the cover layer 9, the high NA lens 10 is positioned. Possibly, layer 9 and lens 10 may be combined into one and the same entity.

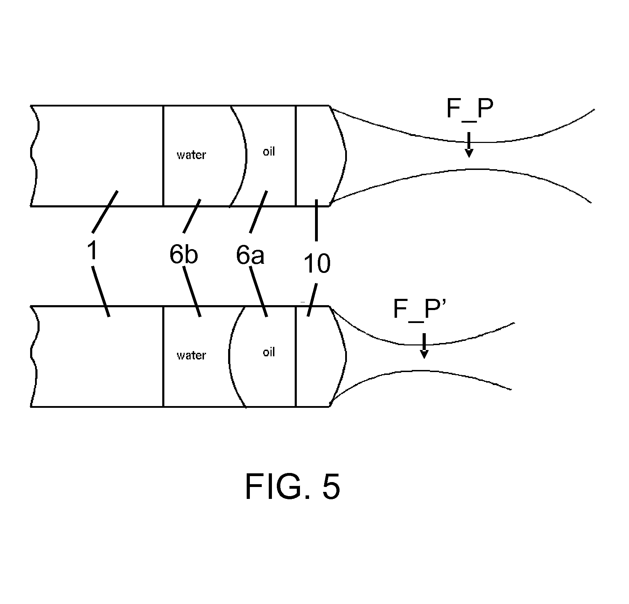

Figure 5 shows two schematic cross-sectional views of an optical image probe 20 according to the present invention having two different settings of the numerical aperture (1NA) of the fluid lens 5. The lens 5 could comprise a water phase and an oil phase to the right.

In the top view, the meniscus of the fluid interface is curved towards the oil phase resulting in a focal point F_P as indicated in the top view of Figure 5. Similarly, in the bottom view, the meniscus of the fluid interface is curved towards the water phase resulting in another focal point F_P' as indicated in the bottom view of Figure 5, the focal point thereby being shifted towards the lens 10. Thus, application of the optical image probe 20 facilitates manipulation of the focal point F_P, in particular so as to enable high lateral resolution during OCT imaging.

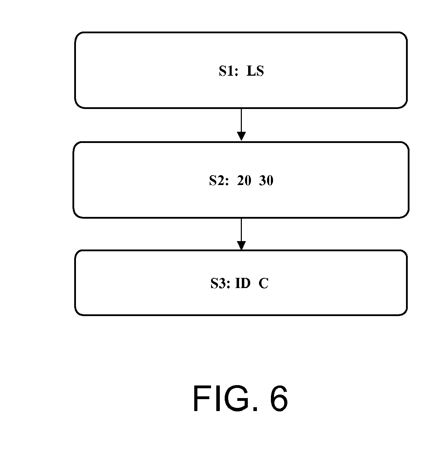

Figure 6 is a schematic flow chart of a method for imaging according to the present invention with an imaging system for optical coherence tomography (OCT), the method comprising:

S1 providing a light source LS,

S2 providing a sample arm 30 optically coupled to the light source, the sample arm having at its distal end an optical image probe 20, and

S3 performing imaging with an imaging detector ID optically coupled via C to the light source LS and the sample arm 30.

The invention can be implemented by means of hardware, software, firmware or any combination of these. The invention or some of the features thereof can also be implemented as software running on one or more data processors and/or digital signal processors.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. An optical image probe (20), the probe comprising:

   - an optical light guide (1) arranged for a transmitting single-mode light,
   - a fluid lens (5) positioned at an end portion of the optical light guide, the fluid lens having a changeable numerical aperture (1NA), and
   - a lens (10) positioned in front of the fluid lens, the lens having a fixed numerical aperture (2NA).

2. The probe according to claim 1, wherein the fixed numerical aperture (2NA) is larger than a maximum value of the changeable numerical aperture (1NA).

**3.** The probe according to claim 1, wherein the numerical aperture (2NA) of the lens (10) and the numerical aperture (1NA) of the fluid lens (5) in combination facilitate a lateral resolution of the optical probe below 10 micrometer, preferably below 8 micrometer, or more preferably below 6 micrometer.

**4.** The probe according to claim 1, wherein the lens (10) in front of the fluid lens (5) has a numerical aperture (NA) of at least 0.3, at least 0.5, or at least 0.7

**5.** The probe according to claim 1, wherein the fluid lens (5) is an electrowetting lens comprising two immiscible fluids (6a, 6b).

**6.** The probe according to claim 5, wherein the electrowetting lens (5) has an asymmetric electrode configuration (7, 8) so as to enable tilting of the meniscus formed between the two immiscible fluids (6a, 6b).

**7.** The probe according to claim 1, the probe (20) being arranged for optical coherence tomography (OCT), confocal imaging, spectroscopy imaging, or fluorescent imaging.

**8.** The probe according to claim 1, wherein the optical image probe (20) is arranged to form part of an endoscope, a catheter or a needle.

**9.** The probe according to claim 1, wherein the probe is arranged for optical coherence tomography (OCT) and the probe has a corresponding reference arm (101), the reference arm having substantially the same optical properties as the optical image probe at least with respect to optical dispersion and intensity loss.

**10.** An imaging system for optical coherence tomography (OCT), the system comprising:

- a light source (LS),
- a sample arm (30) optically coupled to the light source, the sample arm having at its distal end an optical image probe (20) according to claim 1, and
- an imaging detector (ID) optically coupled (C) to the light source (LS) and the sample arm (30).

**11.** The imaging system according to claim 10, wherein the system is arranged for time-domain optical coherence tomography (TD-OCT), the imaging detector further being optically coupled to a reference arm (101) with a displaceable reference mirror (102), the numerical aperture (1NA) of the fluid lens (5) being dependent on the position of the reference mirror (102) during axial scans of the imaging system.

**12.** The imaging system according to claim 10, wherein the system is arranged for frequency-domain optical coherence tomography (FD-OCT), the light source (LS) having a tunable wavelength, the numerical aperture (1NA) of the fluid lens being dependent on the wavelength ($\lambda$) of the light source.

**13.** The imaging system according to claim 11 or 12, wherein the system is adapted to adjust the numerical aperture (1NA) of the fluid lens (5) so as to keep the sampling point (SP) within the focal dept (F) of the imaging system.

**14.** A method for imaging with an imaging system for optical coherence tomography (OCT), the method comprising:

- providing a light source (LS),
- providing a sample arm (30) optically coupled to the light source, the sample arm having at its distal end an optical image probe (20) according to claim 1, and
- performing imaging with an imaging detector (ID) optically coupled (C) to the light source (LS) and the sample arm (30).

20:

FIG. 1

FIG. 2

FIG. 3

EP 2 070 468 A1

FIG. 4

FIG. 5

S1: LS

S2: 20 30

S3: ID C

FIG. 6

**European Patent Office**

**EUROPEAN SEARCH REPORT**

Application Number

EP 07 12 3234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/041376 A (GEN HOSPITAL CORP [US]; TEARNEY GUILLERMO J [US]; YELIN DVIR [US]; BOU) 12 April 2007 (2007-04-12) <br> * paragraphs [0091], [0093] * <br> * paragraph [0106] * <br> * paragraphs [0112] - [0114] * <br> * paragraph [0126] * <br> * paragraphs [0139], [0140] * <br> * figures 4,9,10,24,26 * <br> ----- | 1-14 | INV. <br> A61B5/00 <br> G02B3/12 |
| X | PANOMSAK MEEMON ET AL: "Dynamic focus catheter design for endoscopic optical coherence tomography" <br> LASERS AND ELECTRO-OPTICS SOCIETY, 2007. LEOS 2007. THE 20TH ANNUAL ME ETING OF THE IEEE, IEEE, PI, <br> 21 October 2007 (2007-10-21), - 25 October 2007 (2007-10-25) pages 9-10, XP031160422 <br> ISBN: 978-1-4244-0924-2 <br> * the whole document * <br> ----- | 1-14 | |
| A | US 2004/254474 A1 (SEIBEL ERIC [US] ET AL) 16 December 2004 (2004-12-16) <br> * paragraphs [0011] - [0013] * <br> * paragraph [0052] * <br> * paragraph [0084] * <br> * figures 8,16A,16B * <br> ----- | 1,5, 7-10,14 | TECHNICAL FIELDS SEARCHED (IPC) <br><br> A61B <br> G02B |
| A | WO 2007/085658 A (VARIOPTIC SA [FR]; CRAEN PIERRE [FR]; TALLARON NICOLAS [FR]) 2 August 2007 (2007-08-02) <br> * page 1, lines 4-7 * <br> * page 13, line 7 - page 14, line 3 * <br> * page 17, line 21 - page 18, line 7 * <br> * figure 11 * <br> ----- <br> -/-- | 1,5,7,8, 14 | |

The present search report has been drawn up for all claims

| Place of search | Date of completion of the search | Examiner |
|---|---|---|
| Berlin | 27 May 2008 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

## EUROPEAN SEARCH REPORT

Application Number

EP 07 12 3234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/033326 A (WELCH ALLYN INC [US]; KRAUTER ALLAN I [US]; NEWMAN RICHARD W [US]; LIA) 22 March 2007 (2007-03-22)<br>* paragraphs [0100] - [0102] *<br>* paragraph [0150] *<br>* figures 16,33 *<br>----- | 1,5-8,14 | |
| A | US 2007/236699 A1 (CHOU CHIEN [TW] ET AL) 11 October 2007 (2007-10-11)<br>* paragraphs [0012], [0016] - [0018] *<br>* figures 1-3 *<br>----- | 1,7,9-14 | |

**TECHNICAL FIELDS SEARCHED (IPC)**

The present search report has been drawn up for all claims

| Place of search | Date of completion of the search | Examiner |
|---|---|---|
| Berlin | 27 May 2008 | Völlinger, Martin |

EPO FORM 1503 03.82 (P04C01)

**ANNEX TO THE EUROPEAN SEARCH REPORT
ON EUROPEAN PATENT APPLICATION NO.**

EP 07 12 3234

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2008

| Patent document cited in search report | | | Publication date | Patent family member(s) | | Publication date |
|---|---|---|---|---|---|---|
| WO 2007041376 | A | | 12-04-2007 | AU 2006299659 A1 | | 12-04-2007 |
| | | | | WO 2007041382 A1 | | 12-04-2007 |
| | | | | WO 2007041412 A1 | | 12-04-2007 |
| | | | | WO 2007038787 A1 | | 05-04-2007 |
| US 2004254474 | A1 | | 16-12-2004 | EP 1768564 A2 | | 04-04-2007 |
| | | | | JP 2008504557 T | | 14-02-2008 |
| | | | | WO 2006004743 A2 | | 12-01-2006 |
| WO 2007085658 | A | | 02-08-2007 | NONE | | |
| WO 2007033326 | A | | 22-03-2007 | NONE | | |
| US 2007236699 | A1 | | 11-10-2007 | NONE | | |

EPO FORM P0459

For more details about this annex : see Official Journal of the European Patent Office, No. 12/82

**EP 2 070 468 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050113912 A

**Non-patent literature cited in the description**

- Handbook of Optical Coherence Tomography. 2002, 6-8